# EUROPEAN PATENT SPECIFICATION

(11) **EP 2 886 544 B1**
(45) Date of publication and mention of the grant of the patent: **21.11.2018**
(21) Application number: 14198455.9
(22) Date of filing: 17.12.2014
(51) Int. Cl.: C07D 487/04

(54) **Process for the preparation of crystalline sitagliptin fumarate**
Verfahren zur Herstellung von kristallinem Sitagliptin-Fumarat
Procédé de préparation de fumarate de sitagliptine sous forme cristalline

(30) Priority: 17.12.2013 IN 3677DE2013
(43) Date of publication of application: 24.06.2015
(73) Proprietor: Sun Pharmaceutical Industries Limited, 400 063 Mumbai MAH (IN)
(72) Inventor: Shah, Jigar Bhaskarbhai, 392001 Bharuch, Gujarat (IN); Kanawade, Sandeep T., 422604 Ahmednagar, Maharashtra (IN); Jayachandra, Suresh Babu, 500072 Hyderabad, Andhra Pradesh (IN)
(74) Representative: Cronin, Brian Harold John

(56) References cited:
- WO-A1-2013/084210
- WO-A2-2009/085990
- WO-A2-2010/000469
- US-A1- 2010 249 140
- KIM DOOSEOP ET AL: "(2R)-4-Oxo-4-[3-(Trifluoromethyl)-5,6-dih ydro[1,2,4]triazolo[4,3- a]pyrazin-7(8H)-yl]-1-(2,4,5-trifluorophen yl)butan-2-amine: A Potent, Orally Active Dipeptidyl Peptidase IV Inhibitor for the Treatment of Type 2 Diabetes", JOURNAL OF MEDICINAL CHEMISTRY, AMERICAN CHEMICAL SOCIETY, US, vol. 48, 1 January 2005 (2005-01-01), pages 141-151, XP002529729, ISSN: 0022-2623, DOI: 10.1021/JM0493156 [retrieved on 2004-12-13]

## Description

### Field of the Invention

The present invention provides a process for the preparation of a crystalline sitagliptin fumarate.

### Background of the Invention

Sitagliptin phosphate monohydrate of Formula A, an orally-active inhibitor of the dipeptidyl peptidase-4 (DPP-4) enzyme, chemically designated as 7-[(3*R*)-3-amino-1-oxo-4-(2,4,5-trifluorophenyl)butyl]-5,6,7,8-tetrahydro-3-(trifluoromethyl)-1,2,4-triazolo[4,3-*a*]pyrazine phosphate (1:1) monohydrate, is indicated as an adjunct to diet and exercise to improve glycemic control in adults with type 2 diabetes mellitus.

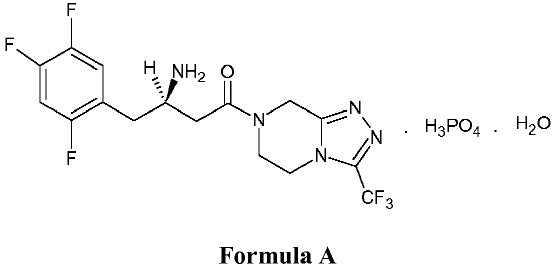

PCT Publication No. WO 2009/085990 provides a process for the preparation of crystalline sitagliptin fumarate which involves heating a mixture of sitagliptin and isopropanol to about 80°C to obtain a clear solution. Fumaric acid was added to the solution, the mixture was refluxed for 2 hours, cooled to 30°C, stirred for 21 hours, filtered, washed with isopropanol, and dried under reduced pressure at about 45°C for 2 hours to obtain the crystalline sitagliptin fumarate.

PCT Publication No. WO 2010/000469 provides a process for the preparation of sitagliptin fumarate Form I and sitagliptin fumarate Form II.

PCT Publication No. WO 2010/117738 provides a process for the preparation of crystalline Form F1 of sitagliptin fumarate, crystalline Form F2 of sitagliptin fumarate, and a mixture of crystalline Forms F1 and F2 of sitagliptin fumarate.

U.S. Publication No. 2012/0142693 provides a process for the preparation of crystalline Form A, crystalline Form B, and crystalline Form C of sitagliptin fumarate.

Journal of Medicinal Chemistry, 48(1):141-151 (2005), provides a process for the preparation of sitagliptin fumarate. The process involves dissolving sitagliptin and fumaric acid in ethanol, stirring for 2 minutes followed by heating gently to get a cloudy solution. To the cloudy solution, anhydrous diethyl ether was added dropwise, stirred at room temperature for 5 minutes, filtered, washed with diethyl ether, and dried in vacuum at 100°C for 30 minutes to obtain sitagliptin fumarate.

The present inventors have found that sitagliptin fumarate obtained by the above mentioned process contained more than 0.15% of individual impurities at RRT 0.54 and RRT 0.56, respectively, as measured by HPLC. Therefore, there exists a need in the art to control the level of the impurities at RRT 0.54 and RRT 0.56, respectively, in sitagliptin fumarate.

### Summary of Invention

The inventors of the present invention surprisingly found that impurities in crystalline sitagliptin fumarate formed at RRT 0.54 and RRT 0.56, respectively, can be substantially removed by treating a mixture of sitagliptin fumarate in methanol with isopropanol. The process is effective to reduce the impurities at RRT 0.54 and RRT 0.56 present in crystalline sitagliptin fumarate and is suitable for commercial scale.

The present invention provides a process for the preparation of crystalline sitagliptin fumarate, comprising the steps of:
a) treating fumaric acid with sitagliptin in methanol;
b) partially concentrating the mixture of step a);
c) treating the partially concentrated mixture of step b) with isopropanol at a temperature of 20°C to 35°C; and
d) isolating crystalline sitagliptin fumarate.

### Detailed Description of Invention

In the present invention, "treating" includes adding, dissolving, slurrying, stirring, and combinations thereof.

In one embodiment, step a) includes the addition of sitagliptin fumarate to methanol, or using an existing mixture from a previous processing step of sitagliptin fumarate in methanol. The molar ratio of sitagliptin to fumaric acid is about 1:0.5 to 1:1.5.

The addition of isopropanol is preferably performed drop-wise for a time period of 30 minutes to 1 hour. After the isopropanol has been added, the resultant mixture is stirred at a temperature of about 25°C to about 30°C for about 5 hours to 15 hours.. Partially concentrating the mixture in step b) involves removal of a solvent from the reaction mixture by about 10% to about 90%, more preferably by about 10% to about 75%, and most preferably by about 10% to about 50%. The removal of the solvent from the reaction mixture can be performed by techniques such as evaporation or evaporation under vacuum. Evaporation can be carried out at a temperature of about 35°C to 50°C, preferably under vacuum.

In one embodiment, step a) is performed at a temperature of from about 25°C to about 60°C. The molar ratio of sitagliptin to fumaric acid is also about 1:0.5 to 1:1.5.

In another embodiment, step b) involves removal of a solvent from the reaction mixture by about 10% to about 90%, more preferably by about 10% to about 75%, and most preferably by about 10% to about 50%. The removal of the solvent from the reaction mixture can be performed by techniques such as evaporation or evaporation under vacuum. Evaporation can be carried out at a temperature of about 35°C to 50°C, preferably under vacuum.

Preferably, in step c) the isopropanol is added at about 25°C to about 30°C. The addition is preferably performed drop-wise for about 30 minutes to about 1 hour. After completion of the addition, the resultant mixture is stirred at a temperature of about 25°C to about 30°C for about 5 hours to about 15 hours.

In another embodiment, step d) can be performed by evaporation, evaporation under vacuum, cooling, extraction, washing, precipitation, filtration, filtration under vacuum, decantation, centrifugation, or combinations thereof.

Crystalline sitagliptin fumarate obtained by the process of this invention contains less than 0.10% of individual impurities at RRT 0.54 or RRT 0.56, respectively, as measured by HPLC. In a preferred embodiment, crystalline sitagliptin fumarate obtained by the process of this invention contains less than 0.08% of individual impurities at RRT 0.54 or RRT 0.56, respectively, as measured by HPLC. In a most preferred embodiment, crystalline sitagliptin fumarate obtained by the process of this invention contains less than 0.05% of individual impurities at RRT 0.54 or RRT 0.56, respectively, as measured by HPLC. In another preferred embodiment, crystalline sitagliptin fumarate obtained by the process of this invention contains about 0.01% to about 0.08% of individual impurities at RRT 0.54 or RRT 0.56 as measured by HPLC.

HPLC purity was determined by using a Nucleodur® C₁₈ Gravity column, 5µm (250 mm x 4.6 mm) having the following parameters:
Flow rate: 0.5 mL/minute
Detector: UV at 210 nm
Injection volume: 20 µL
Run time: 70 minutes
Buffer: Potassium dihydrogen phosphate pH 7.00
Organic phase: 40:60 Acetonitrile:Methanol
Mobile phase A: 90:10 Buffer:Acetonitrile
Mobile phase B: 90:10 Organic phase:Buffer

While the present invention has been described in terms of its specific embodiments, certain modifications and equivalents will be apparent to those skilled in the art and are intended to be included within the scope of the present invention.

### EXAMPLES

### Reference Example 1: Preparation of crystalline sitagliptin fumarate (as per the method provided in Journal of Medicinal Chemistry, Vol. 48(1):141-151 (2005))

Sitagliptin free base (100 g), fumaric acid (17.13 g), and ethanol (302 mL) were charged into a reactor. The reaction mixture was heated to 55°C to 60°C. The reaction mixture was stirred until the reaction mixture became cloudy and was then cooled to 30°C. Diethyl ether (500 mL) was charged in the reactor over about 20 minutes to about 30 minutes. The reaction mixture was filtered, then the solid was washed with diethyl ether (300 mL). The solid was dried under vacuum at 100°C for 30 minutes to obtain the title compound.
Yield: 98 g
HPLC purity: 99.08%
Impurity at RRT 0.54 by HPLC: 0.40%
Impurity at RRT 0.56 by HPLC: 0.41%

### Example 1: Preparation of crystalline sitagliptin fumarate

Sitagliptin free base (100 g) and methanol (0.5 L) were charged into a reactor and stirred for 5 minutes at 26°C to 28°C. Fumaric acid (17.13 g) was charged in the reactor at 26°C to 28°C while stirring and heated to 50°C to 55°C. The reaction mixture was stirred for about 4 hours at 50°C to 55°C and then cooled to 25°C to 30°C. Isopropanol (1 L) was slowly added over about 30 minutes at 26°C to 28°C. The reaction mixture was stirred at 26°C to 28°C for about 15 hours, then filtered, then the solid obtained was washed with isopropanol (200 mL). The solid was dried under vacuum at 100°C for 30 minutes to obtain the title compound.
Yield: 81 g
HPLC purity: 99.93%
Impurity at RRT 0.54 by HPLC: 0.03%
Impurity at RRT 0.56 by HPLC: 0.03%

### Example 2: Preparation of crystalline sitagliptin fumarate

Sitagliptin free base (100 g) and methanol (1 L) were charged into a reactor and stirred for 5 minutes at 26°C to 28°C. Fumaric acid (28.5 g) was charged into the reactor at 26°C to 28°C while stirring and heated to 55°C to 60°C. The reaction mixture was stirred for 30 minutes at 55°C to 60°C and then cooled to 40°C. The reaction mixture was concentrated (up to 10%). Isopropanol (500 mL) was slowly added over about 30 minutes at 26°C to 28°C. The reaction mixture was stirred at 26°C to 28°C for 12 hours, then filtered, then the solid obtained was washed with isopropanol (200 mL). The solid was dried under vacuum at 45°C to 50°C for 16 hours to obtain the title compound.
Yield: 106 g
HPLC purity: 99.49%
Impurity at RRT 0.54 by HPLC: 0.06%
Impurity at RRT 0.56 by HPLC: 0.04%

### Example 3: Preparation of crystalline sitagliptin fumarate

Sitagliptin free base (100 g) and methanol (1 L) were charged into a reactor and stirred for 5 minutes at 26°C to 28°C. Fumaric acid (28.5 g) was charged into the reactor at 26°C to 28°C while stirring and heated to 55°C to 60°C. The reaction mixture was stirred for 4 hours at 55°C to 60°C and then cooled to 40°C. The reaction mixture was concentrated to the maximum extent possible. Isopropanol (500 mL) was slowly added over about 30 minutes at 26°C to 28°C. The reaction mixture was stirred at 26°C to 28°C for about 15 hours, then filtered. The solid obtained was washed with isopropanol (200 mL), then dried under vacuum at 100°C for about 30 minutes to obtain the title compound.
Yield: 100 g
HPLC purity: 99.49%
Impurity at RRT 0.54 by HPLC: 0.12%
Impurity at RRT 0.56 by HPLC: 0.13%

## Claims

1. A process for the preparation of crystalline sitagliptin fumarate, comprising the steps of:
a) treating fumaric acid with sitagliptin in methanol;
b) partially concentrating the mixture of step a);
c) treating the concentrated mixture of step b) with isopropanol at a temperature of 20°C to 35°C; and
d) isolating crystalline sitagliptin fumarate.

2. The process according to claim 1, wherein step a) is performed at a temperature of 25°C to 60°C.

3. The process according to claim 1, wherein the molar ratio of the sitagliptin to the fumaric acid is 1:0.5 to 1:1.5.

4. The process according to claim 1, wherein step b) involves removal of a solvent from the reaction mixture by 10% to 90%.

5. The process according to claim 1, wherein step b) involves removal of a solvent from the reaction mixture by 10% to 75%.

6. The process according to claim 1, wherein step b) involves removal of a solvent from the reaction mixture by 10% to 50%.

7. The process according to claim 4, claim 5, or claim 6, wherein the removal of the solvent from the reaction mixture is performed by evaporation at a temperature of 35°C to 50°C under vacuum.

8. The process according to claim 1, wherein step c) involves adding, dissolving, slurrying, stirring, or a combination thereof.

9. The process according to claim 8, wherein the addition of isopropanol is performed dropwise for a time period of 30 minutes to 1 hour.

10. The process according to claim 8, wherein stirring is performed at a temperature of 25°C to 30°C for a time period of 5 hours to 15 hours.

11. The process according to claim 1, wherein the crystalline sitagliptin fumarate obtained contains about 0.01% to about 0.08% of individual impurity at RRT 0.54 or about 0.01% to about 0.08% of individual impurity at RRT 0.56 as measured by HPLC.

## Patentansprüche

1. Verfahren zur Herstellung von kristallinem Sitagliptinfumarat, das folgende Phasen umfasst:
a) Behandeln von Fumarsäure mit Sitagliptin in Methanol;
b) teilweises Konzentrieren der Mischung von Phase a);
c) Behandeln der konzentrierten Mischung von Phase b) mit Isopropanol bei einer Temperatur von 20°C bis 35°C; und
d) Isolierung des kristallinen Sitagliptinfumarats.

2. Verfahren nach Anspruch 1, wobei Phase a) bei einer Temperatur von 25°C bis 60°C durchgeführt wird.

3. Verfahren nach Anspruch 1, wobei das Molverhältnis des Sitagliptins zu der Fumarsäure 1:0,5 bis 1:1,5 beträgt.

4. Verfahren nach Anspruch 1, wobei Phase b) die Entfernung eines Lösungsmittels aus der Reaktionsmischung um 10% bis 90% umfasst.

5. Verfahren nach Anspruch 1, wobei Phase b) die Entfernung eines Lösungsmittels aus der Reaktionsmischung um 10% bis 75% umfasst.

6. Verfahren nach Anspruch 1, wobei Phase b) die Entfernung eines Lösungsmittels aus der Reaktionsmischung um 10% bis 50% umfasst.

7. Verfahren nach Anspruch 4, Anspruch 5 oder Anspruch 6, wobei die Entfernung des Lösungsmittels aus der Reaktionsmischung durch Verdampfung bei einer Temperatur von 35°C bis 50°C unter Vakuum durchgeführt wird.

8. Verfahren nach Anspruch 1, wobei Phase c) Zugabe, Auflösung, Aufschlämmung, Rühren oder eine Kombination davon umfasst.

9. Verfahren nach Anspruch 8, wobei die Zugabe von Isopropanol tropfenweise für eine Zeit von 30 Minuten bis 1 Stunde durchgeführt wird.

10. Verfahren nach Anspruch 8, wobei das Rühren bei einer Temperatur von 25°C bis 30°C für eine Zeit von 5 Stunden bis 15 Stunden durchgeführt wird.

11. Verfahren nach Anspruch 1, wobei das erhaltene kristalline Sitagliptinfumarat etwa 0,01% bis etwa 0,08% individuelle Verunreinigung bei RRT 0,54 oder etwa 0,01% bis etwa 0,08% individuelle Verunreinigung bei RRT 0,56 enthält, wie mittels HPLC gemessen.

## Revendications

1. Procédé pour la préparation de fumarate de sitagliptine cristallin, comprenant les phases de :
a) traiter de l'acide fumarique avec de la sitagliptine en méthanol ;
b) concentrer partiellement le mélange de la phase a) ;
c) traiter le mélange concentré de la phase b) avec de l'isopropanol à une température de 20°C à 35°C ; et
d) isoler le fumarate de sitagliptine cristallin.

2. Procédé selon la revendication 1, où la phase a) est accomplie à une température de 25°C à 60°C.

3. Procédé selon la revendication 1, où le rapport molaire sitagliptine sur acide fumarique est 1:0,5 à 1:1,5.

4. Procédé selon la revendication 1, où la phase b) implique l'enlèvement de 10% à 90% d'un solvant du mélange de réaction.

5. Procédé selon la revendication 1, où la phase b) implique l'enlèvement de 10% à 75% d'un solvant du mélange de réaction.

6. Procédé selon la revendication 1, où la phase b) implique l'enlèvement de 10% à 50% d'un solvant du mélange de réaction.

7. Procédé selon la revendication 4, revendication 5, ou revendication 6, où l'enlèvement du solvant du mélange de réaction est accompli par évaporation à une température de 35°C à 50°C sous vide.

8. Procédé selon la revendication 1, où la phase c) implique l'addition, la dissolution, la suspension, l'agitation, ou une combinaison de cela.

9. Procédé selon la revendication 8, où l'addition d'isopropanol est accomplie goutte à goutte pendant une période de temps de 30 minutes à 1 heure.

10. Procédé selon la revendication 8, où l'agitation est accomplie à une température de 25°C à 30°C pendant une période de temps de 5 heures à 15 heures.

11. Procédé selon la revendication 1, où le fumarate de sitagliptine cristallin obtenu contient environ 0,01% à environ 0,08% d'impureté individuelle à RRT 0,54 ou environ 0,01% à environ 0,08% d'impureté individuelle à RRT 0,56 comme mesurée par HPLC.
